# EUROPÄISCHE PATENTSCHRIFT

(11) **EP 3 047 807 B1**
(45) Veröffentlichungstag und Bekanntmachung des Hinweises auf die Patenterteilung: **19.09.2018**
(21) Anmeldenummer: 15201259.7
(22) Anmeldetag: 18.12.2015
(51) Int. Cl.: A61B 17/16, A61B 90/92, A61F 2/36, A61F 2/30

(54) **MEDIZINISCHES INSTRUMENTARIUM UND IMPLANTATIONSSET**
MEDICAL EQUIPMENT AND IMPLANTATION SET
INSTRUMENT MEDICAL ET JEU D'IMPLANTATION

(30) Priorität: 18.12.2014 DE 102014119083
(43) Veröffentlichungstag der Anmeldung: 27.07.2016
(73) Patentinhaber: AESCULAP AG, 78532 Tuttlingen (DE)
(72) Erfinder: Reu, Gerhard, 78532 Tuttlingen (DE); Bader, Uwe, 78532 Tuttlingen (DE)
(74) Vertreter: Hoeger, Stellrecht & Partner Patentanwälte mbB

(56) Entgegenhaltungen:
- EP-A1- 2 027 833
- WO-A1-96/17553
- DE-U1- 29 816 064
- US-A- 5 019 108
- US-A- 5 766 261
- US-A1- 2012 259 338
- US-A1- 2014 276 850

## Beschreibung

Die vorliegende Erfindung betrifft ein medizinisches Instrumentarium, insbesondere zum Implantieren eines Hüftgelenkschafts, umfassend ein Raspelinstrument mit einem eine Längsrichtung definierenden Raspelschaft, wobei am Raspelschaft mindestens zwei Tiefenanschläge angeordnet oder ausgebildet sind, wobei mindestens zwei der mindestens zwei Tiefenanschläge mindestens eine Nut umfassen.

Ferner umfasst die vorliegende Erfindung ein Implantationsset umfassend ein modulares Implantatset mit mindestens zwei unterschiedlichen Implantatteilen und ein medizinisches Instrumentarium, insbesondere zum Implantieren eines Hüftgelenkschafts, umfassend ein Raspelinstrument mit einem eine Längsrichtung definierenden Raspelschaft, wobei am Raspelschaft mindestens zwei Tiefenanschläge angeordnet oder ausgebildet sind, wobei jeder der mindestens zwei Tiefenanschläge mindestens eine Tiefenanschlagfläche umfasst.

Künstliche Hüftgelenke, sogenannte Hüftgelenkendoprothesen, werden häufig bei Patienten eingesetzt, deren Hüftgelenke übermäßige Abnutzungserscheinungen zeigen. Andere Indikationen sind die sogenannte Coxa vara und die sogenannte Coxa valga. Bei der Coxa vara, der "auswärtsgebogene Hüfte", spricht man, wenn der sogenannte CCD-Winkel (Centrum-Collum-Diaphysen-Winkel), also der Winkel zwischen Oberschenkelhals und Schaft des Oberschenkelknochens, unter 120° liegt. Von einer "normalen Hüfte" spricht man, also von einer sogenannten Coxa norma, wenn der CCD-Winkel etwa 125° beträgt. Das Gegenteil einer Coxa vara ist die Coxa valga, die häufig bei Kleinkindern und Neugeborenen auftritt und eine Vergrößerung des CCD-Winkels auf mehr als 135° darstellt. Die Abweichungen von der Norm ergeben sich aufgrund von Wachstum - bei Neugeborenen sind Coxa valga-Situationen normal - und aufgrund pathologischer Änderungen. Beispielsweise bildet sich bei Senioren häufig eine Coxa vara aus. Aber auch aufgrund nationaler Unterschiede gibt es große Abweichungen in den Geometrien im proximalen Femurbereich.

Für die endoprothetische Versorgung einer Hüfte sind besonders unterschiedliche mediale Konturen des Femurkanals, unterschiedliche Winkel des Schenkelhalses und damit verbunden auch die daraus resultierenden Positionen des Kopfmittelpunktes der Hüftgelenkendoprothesen von Bedeutung. Um diese drei oben beschriebenen Klassen, Coxa valga, Coxa norma und Coxa vara, entsprechend behandeln zu können, muss die sichere Fixierung des Implantates im Femurkanals sichergestellt werden. Dabei ist abhängig von der Art der Fixierung des Implantates im Femurkanal die mediale Anlage des Prothesenschafts an der Kortikalis des Femurs sehr wichtig. Daher ist ein Implantatsystem immer für eine der drei genannten Gruppen optimiert und kann zur Versorgung der anderen Gruppen nicht oder nur in Ausnahmefällen genutzt werden. Ferner ist bei den drei genannten Gruppen auch die Position des Rotationszentrums des Gelenks charakteristisch. Im Vergleich zu einem Standardfemur weist ein valgischer Femur eher ein kleines und ein varischer Femur einen eher großen Offset auf. Diese Bandbreite und vor allem das Zusammenspiel aus spezifischer medialer Anlage und Position des Kopfmittelpunkts sind mit den üblichen Systemen nicht abzudecken.

Ein Problem ist, dass bei der Implantation der unterschiedlichen Hüftgelenkendoprothesen, die in Abhängigkeit der pathologischen Situation des Patienten implantiert werden, umfangreiche Instrumentarien benötigt werden.

Aus der US 5,019,108 ist ein modulares Implantat bekannt. Ein distales Fräswerkzeug zur Verwendung bei einem orthopädischen chirurgischen Eingriff zum Iimplantieren einer Revisionshüftgelenkendoprothese ist in der US 2012/ 0259338 A1 beschrieben. Eine Reibahle für den Femur mit modularen Probekomponenten ist in der US 5,766,216 offenbart. Die DE 298 16 064 U1 betrifft Raspeln zum Aushöhlen und Anpassen von Knochenhöhlen an hierin einzusetzende Hohlschaftprothesen. Ein chirurgisches Instrument zur Bearbeitung des Femurs ist aus der US 2014/0276850 A1 bekannt. Die WO 96/17553 A1 betrifft modulare Humerus-Prothesen und modulare Instrumente zum Präparieren eines Humerus. In der EP 2 027 833 A1 ist eine prothetische Probehalskomponente beschrieben.

Daher ist es eine Aufgabe der vorliegenden Erfindung, die Implantation einer Hüftgelenkendoprothese und den Einsatz eines Implantationssets der eingangs beschriebenen Art zu vereinfachen.

Diese Aufgabe wird bei einem medizinischen Instrumentarium der eingangs beschriebenen Art erfindungsgemäß dadurch gelöst, dass die Nuten am Raspelschaft parallel oder im Wesentlichen parallel zu einer in proximaler Richtung weisenden Endfläche des Raspelschafts verlaufen, dass das Instrumentarium ein am Raspelschaft angeordnetes oder ausgebildetes Kopplungselement zum Koppeln des Raspelschafts mit einem Griffelement umfasst, dass das Kopplungselement von der Endfläche in proximaler Richtung oder im Wesentlichen in proximaler Richtung weisend abstehend angeordnet oder ausgebildet ist und dass die durch die mindestens zwei Tiefenanschläge definierten Tiefenanschlagflächen unterschiedliche Oberflächenstrukturen aufweisen oder unterschiedlich eingefärbt sind.

Ein derart ausgebildetes Instrumentarium ermöglicht es, mit nur einem Raspelinstrument Femurknochen von Patienten zur Implantation eines Hüftgelenksschafts vorzubereiten, die unterschiedliche pathologische Situationen an der Hüfte aufweisen. Zum Beispiel kann die Konstruktion der medialen Geometrie der Prothesenschäfte über einen Bogen erfolgen, der bei allen drei Varianten von Prothesenschäften zur Behandlung von Coxa vara, Coxa norma und Coxa valgus den gleichen Radius besitzt. Aufgrund der unterschiedlichen Höhen des Kreismittelpunktes des Schaftbogens wird jedoch, trotz gleichem Radius, eine unterschiedliche mediale Anlage erzeugt. Ist die übrige Geometrie des Prothesenschafts unterhalb einer Resektionslinie bei allen drei Varianten identisch, kann prinzipiell mit demselben Raspelinstrument das Implantatbett für alle drei Arten von Prothesenschäften präpariert werden. Voraussetzung ist lediglich, dass der Raspelschaft um den Wert, um den die Mittelpunkte der medialen Radien der Prothesenschäfte gegeneinander verschoben sind, tiefer oder nicht so tief in den Femurkanal eingeschlagen wird, beispielsweise 5 mm tiefer oder 5 mm weniger tief. Um sicher zu stellen, dass der Raspelschaft nur so tief eingeschlagen wird, dass der jeweils zu implantierende Prothesenschaft medialseitig optimal am Femur anliegt, sind mindestens zwei Tiefenanschläge am Raspelschaft angeordnet oder ausgebildet. Diese ermöglichen es beispielsweise, eine Einschlagtiefe zu begrenzen oder dem Operateur anzuzeigen, wie tief er den Raspelschaft bereits in den Femurkanal eingeschlagen hat. Das Vorsehen der mindestens zwei Tiefenschläge am Raspelschaft ermöglicht es insbesondere, mit nur einem einzigen Raspelinstrument mindestens zwei, vorzugsweise drei unterschiedliche Prothesenschäfte optimal zu implantieren. Vorteilhafterweise umfassen mindestens zwei der mindestens zwei Tiefenanschläge mindestens eine Nut. Beispielsweise können zwei Nuten und die Endfläche insgesamt drei Tiefenanschläge bilden. Zudem kann jeder Tiefenanschlag auch zwei oder mehr Nuten umfassen. Beispielsweise bei einem im Querschnitt rechteckigen Raspelschaft können pro Tiefenanschlag insgesamt bis zu vier Nuten auf vier Außenflächen ausgebildet werden, die zusammen insbesondere eine Ringnut bilden können und zusammen einen einzigen Tiefenanschlag definieren. Vorteilhaft ist es, dass die mindestens eine Nut am Raspelschaft parallel oder im Wesentlichen parallel zu einer in proximaler Richtung weisenden Endfläche des Raspelschafts verläuft. Beispielsweise dann, wenn die Endfläche selbst einen Tiefenanschlag definiert, können in der beschriebenen Weise Nuten parallel oder im Wesentlichen parallel zur Endfläche ausgebildet werden und so einem Operateur auf einfache Weise einen Abstand zur Endfläche anzeigen. Günstig ist es, dass die durch die mindestens zwei Tiefenanschläge definierten Tiefenanschlagflächen unterschiedliche Oberflächenstrukturen aufweisen oder unterschiedlich eingefärbt sind. Insbesondere ermöglichen Farben einem Operateur auf einfache Weise eine Unterscheidung der mindestens zwei Tiefenanschlagflächen. So kann er sicher erkennen, wie weit der Raspelschaft bereits in den Femurknochen eingetrieben ist und ob er gegebenenfalls noch weiter eingetrieben werden muss. Ferner ist es vorteilhaft, dass das Instrumentarium ein am Raspelschaft angeordnetes oder ausgebildetes Kopplungselement zum Koppeln des Raspelschafts mit einem Griffelement aufweist. Dies ermöglicht es, mit dem Griffelement, welches vorzugsweise eine Einschlagfläche zum Einschlagen des Raspelschafts in den Femur aufweist, den Raspelschaft einzuschlagen und dann den Raspelschaft und das Griffelement voneinander zu trennen, um beispielsweise einen Prothesenhals mit einem Gelenkkopf auf den Raspelschaft aufzusetzen, um Lage und Position des Gelenkkopfes zu bestimmen. Die ist günstig, um dann die optimale Prothesengröße sowie Halslänge und Gelenkkugeldurchmesser festzulegen. Auf besonders einfache Weise lässt sich das Griffelement mit dem Raspelschaft koppeln, wenn das Kopplungselement in proximaler Richtung oder im Wesentlichen in proximaler Richtung weisend angeordnet oder ausgebildet ist. Das Kopplungselement kann insbesondere in Form eines Kopplungsvorsprungs oder aber auch in Form einer Kopplungsaufnahme ausgebildet sein.

Vorzugsweise umfasst eine in proximaler Richtung weisende Endfläche des Raspelschafts die mindestens eine Tiefenanschlagfläche. Von dieser können optional weitere Elemente abstehen, beispielsweise ein Kopplungselement zum Verbinden des Raspelschafts mit einem Handgriff oder dergleichen. Die Endfläche bildet somit einen Tiefenanschlag, welcher dem Operateur anzeigen kann, wie tief der Raspelschaft bereits in den Femurknochen eingetrieben ist.

Günstig ist es, wenn die mindestens zwei Tiefenanschläge voneinander beabstandet angeordnet oder ausgebildet sind. So können unterschiedliche Einschlagtiefen vorgegeben oder dem Operateur angezeigt werden.

Auf besonders einfache Weise ausbilden lässt sich das Instrumentarium, wenn mindestens einer der mindestens zwei Tiefenanschläge mindestens eine Nut umfasst. Beispielsweise kann eine Nut auf einfache Weise durch Fräsen oder andere spanende Bearbeitungsverfahren auf einer Außenseite des Raspelschafts ausgebildet werden.

Vorteilhafterweise ist die mindestens eine Nut von der Endfläche beabstandet. So kann durch einen Abstand zwischen der Endfläche und der mindestens einen Nut für einen Operateur eine unterschiedliche Einschlagtiefe des Raspelschaft vorgegeben und angezeigt werden.

Auf besonders einfache Weise ausbilden lässt sich das Instrumentarium, wenn die mindestens eine Nut eine Nutlängsrichtung definiert und/oder geradlinig oder im Wesentlichen geradlinig verläuft. Ferner erleichtert diese Ausgestaltung einem Operateur auch den Einsatz des Instrumentariums, da er insbesondere sicher eine Einschlagtiefe erkennen kann durch Vergleich einer Position der sichtbaren Nut und eines proximalen Endes des präparierten Femurknochens.

Damit ein Operateur die mindestens zwei Tiefenanschläge auch während eines chirurgischen Eingriffs gut erkennen kann, ist es günstig, wenn die mindestens eine Nut auf einer Seitenfläche des Raspelschafts ausgebildet ist, welche in einer Richtung quer zur Längsrichtung weist.

Gemäß einer weiteren bevorzugten Ausführungsform der Erfindung kann vorgesehen sein, dass die mindestens eine Nut mindestens eine Nutseitenfläche umfasst und dass die mindestens eine Nutseitenfläche die mindestens eine Tiefenanschlagfläche definiert. Beispielsweise kann eine Nut auch zwei, drei oder mehr Nutseitenflächen umfassen, je nach dem, welche Querschnittsform die Nut aufweist. Insbesondere kann die Nut keilförmig mit zwei Nutseitenflächen oder rechteckig mit drei Nutseitenflächen ausgebildet sein.

Damit ein Operateur eine Einschlagtiefe eines Raspelschafts gut erkennen kann, ist es vorteilhaft, wenn die mindestens eine Nutseitenfläche parallel oder im Wesentlichen parallel zur Endfläche verläuft.

Gemäß einer weiteren bevorzugten Ausführungsform der Erfindung kann vorgesehen sein, dass die mindestens zwei Tiefenanschläge jeweils mit einer individuellen Kennzeichnungsfarbe eingefärbt sind und dass die Kennzeichnungsfarbe entweder blau oder grün oder orange ist. Insbesondere können drei Tiefenanschläge jeweils mit einer der drei Farben eingefärbt sein, die eine eindeutige Unterscheidung der drei Tiefenanschläge ermöglichen. Insbesondere können auch die Tiefenanschlagflächen mit der Kennzeichnungsfarbe in der beschriebenen Weise eingefärbt sein.

Günstigerweise sind drei Tiefenschläge vorgesehen. Drei Tiefenanschläge ermöglichen es, eine Implantation entsprechender Prothesenschäfte zur Behandlung von Patienten mit Coxa vara, Coxa norma und Coxa valga mit nur einem einzigen Raspelinstrument durchzuführen. Dies reduziert die Zahl der verfügbar zu haltenden Instrumente und damit Material- und Aufbereitungskosten.

Die Handhabung des Instrumentariums wird weiter vereinfacht, wenn jeder der mindestens zwei Tiefenanschläge mindestens eine Tiefenanschlagfläche umfasst. Ein Tiefenanschlag kann eine, zwei oder mehr Tiefenanschlagflächen umfassen. Diese kennzeichnen vorzugweise dieselbe Einschlagtiefe für den Raspelschaft.

Günstigerweise umfasst mindestens einer der mindestens zwei Tiefenanschläge zwei Tiefenanschlagflächen. Beispielsweise können die zwei Tiefenanschlagflächen derart angeordnet sein, dass sie auf unterschiedlichen Seiten des Raspelschafts angeordnet oder ausgebildet sind. So kann das Raspelinstrument zur Implantation von Hüftschäften sowohl für ein linkes als auch ein rechtes Hüftgelenks des Patienten einfach und sicher genutzt werden.

Vorteilhaft ist es, wenn die zwei Tiefenanschlagflächen des mindestens einen der mindestens zwei Tiefenanschläge voneinander getrennt angeordnet oder ausgebildet sind. So sind sie beispielsweise gut erkennbar am Raspelschaft auf zwei unterschiedlichen, voneinander weg weisenden Seiten anordenbar oder ausbildbar.

Die Herstellung des Instrumentariums vereinfacht sich insbesondere dadurch, dass die mindestens eine Tiefenanschlagfläche eben oder im Wesentlichen eben ist. Eine ebene Tiefenanschlagfläche ist für einen Operateur zudem besonders gut erkennbar, so dass er den Raspelschaft nur so tief wie erforderlich in den Femurknochen einschlagen kann.

Um die Unterscheidbarkeit der mindestens zwei Tiefenanschläge für den Operateur zu verbessern, ist es vorteilhaft, wenn die Tiefenanschlagflächen der mindestens zwei Tiefenanschläge unterschiedlich breit sind.

Vorteilhafterweise ist die proximalste Tiefenanschlagfläche am breitesten. So ist sie auch dann noch gut zu erkennen, wenn der Raspelschaft maximal tief in den Femurknochen eingeschlagen ist.

Günstig ist es, wenn die distalste Tiefenanschlagfläche breiter ist als eine zwischen der distalsten und der proximalsten Tiefenanschlagfläche angeordnete oder ausgebildete mittlere Tiefenanschlagfläche. So sind die Tiefenanschläge, auch wenn sie relativ dicht beieinander liegen, deutlich zu unterscheiden.

Um eine unterschiedliche Einschlagtiefe am Raspelschaft sicher zu markieren, ist es günstig, wenn die mindestens eine Tiefenanschlagfläche eines der mindestens zwei Tiefenanschläge von der mindestens einen Tiefenanschlagfläche eines anderen der mindestens zwei Tiefenanschläge bezogen auf die Längsrichtung beabstandet ist.

Gemäß einer weiteren bevorzugten Ausführungsform der Erfindung kann vorgesehen sein, dass die mindestens eine Tiefenanschlagfläche eines der mindestens zwei Tiefenanschläge parallel oder im Wesentlichen parallel zu der mindestens einen Tiefenanschlagfläche eines anderen der mindestens zwei Tiefenanschläge verläuft. Parallel zueinander laufende Tiefenanschlagflächen sind als Tiefenanschläge für einen Operateur besonders gut erkennbar.

Günstig ist es, wenn der Raspelschaft in proximaler Richtung weisende Endflächenbereiche aufweist, welche die Tiefenanschlagflächen bilden oder umfassen. Insbesondere können die Tiefenanschlagflächen in proximaler Richtung oder im Wesentlichen in proximaler Richtung weisend angeordnet oder ausgebildet sein. So sind sie für einen Operateur auch dann noch gut erkennbar, wenn der Raspelschaft in den Femurknochen eingeschlagen ist.

Vorzugsweise umfasst der Raspelschaft drei, vier, fünf oder mehr Endflächenbereiche. Beispielsweise können zwei Endflächenbereiche jeweils eine Tiefenanschlagfläche umfassen, die gemeinsam einen Tiefenanschlag bilden.

Besonders einfach wird die Herstellung des Raspelschafts, wenn mindestens zwei Tiefenanschlagflächen Tiefenanschlagflächenebenen definieren, welche quer, insbesondere senkrecht zur Längsrichtung verlaufen.

Günstigerweise ist der Raspelschaft symmetrisch oder im Wesentlichen symmetrisch zu einer Symmetrieebene ausgebildet, welche quer, insbesondere senkrecht, zu mindestens einer der Tiefenanschlagflächen verläuft. Ferner kann die Symmetrieebene eine Längsachse des Raspelschafts enthalten. Diese Ausgestaltung vereinfacht den Aufbau des Raspelschafts. Zudem wird dessen Handhabbarkeit verbessert, da die Tiefenanschlagflächen sowohl bei der Vorbereitung eines linken Femurs als auch bei der Vorbereitung eines rechten Femurs gleich gut für den Operateur erkennbar sind.

Günstigerweise umfasst der Raspelschaft drei Tiefenanschlagflächen, die äquidistant oder im Wesentlichen äquidistant bezogen auf die Längsrichtung angeordnet oder ausgebildet sind. Eine solche Ausgestaltung ist insbesondere dann günstig, wenn die Prothesenschäfte zur Behandlung von Coxa valga, Coxa norma und Coxa vara äquidistante mediale Krümmungsmittelpunkte aufweisen.

Günstig ist es, wenn eine proximale Kopplungselementendfläche des Kopplungselements die am proximalsten verlaufende Tiefenanschlagflächenebene berührt oder distal derselben angeordnet ist. Diese Ausgestaltung ermöglicht es, dass nach Lösen des Griffelements insbesondere die proximalste Tiefenanschlagfläche dasjenige Element ist, das am weitesten in proximaler Richtung vom Raspelschaft absteht. Damit ist der Tiefenanschlag gut erkennbar, das Kopplungselement ist so am wenigsten störend angeordnet beziehungsweise ausgebildet.

Gemäß einer weiteren bevorzugten Ausführungsform der Erfindung kann vorgesehen sein, dass eine Kopplungselementlängsachse des Kopplungselements und mindestens eine der Tiefenanschlagflächenebenen einen Winkel einschließen, welcher kleiner als 90° ist. Insbesondere liegt der Winkel in einem Bereich von 50° bis 80°. Den Winkel im angegeben Bereich zu wählen hat insbesondere den Vorteil, dass temporär ein Probehals mit einem Probegelenkkopf mit dem Kopplungselement verbunden werden kann, die dann in die gewünschte Richtung weisen.

Der Aufbau des Instrumentariums vereinfacht sich weiter, wenn die Kopplungselemente Längsachse parallel zur Symmetrieebene verläuft oder in dieser liegt.

Um die Herstellung des Instrumentariums weiter zu vereinfachen, ist es günstig, wenn der Raspelschaft zwei voneinander weg weisende Seitenflächen aufweist. Diese können insbesondere eben oder im Wesentlichen eben geformt sein. Des Weiteren können sie Schneidelemente tragen, beispielsweise Raspelzähne oder Raspelnuten, mit denen eine Kavität des Femurknochens bearbeitet werden kann.

Vorteilhafterweise verlaufen die zwei Seitenflächen parallel oder im Wesentlichen parallel zur Symmetrieebene. So kann die Kavität des Femurknochens besonders kompakt präpariert werden zur Aufnahme des Prothesenschafts der Hüftgelen kendoprothese.

Um die Einschlagtiefe des Raspelschafts für einen Operateur besonders gut erkennbar zu gestalten, ist es vorteilhaft, wenn eine der Tiefenanschlagflächen mindestens an eine der beiden Seitenflächen angrenzt. Vorzugsweise handelt es sich dabei um die distalste Tiefenanschlagfläche.

Der distalste Tiefenanschlag ist für einen Operateur besonders gut erkennbar, wenn die distalste Tiefenanschlagflächenebene quer, insbesondere senkrecht, zu einer von einer der beiden Seitenflächen definierten Seitenflächenebene verläuft.

Die Handhabbarkeit des medizinischen Instrumentariums lässt sich verbessern, wenn es ein Griffelement zum Koppeln mit dem Kopplungselement umfasst. Das Griffelement kann insbesondere an seinem proximalen Ende eine Einschlagfläche für ein Einschlagwerkzeug aufweisen, um den Raspelschaft in die Kavität des Femurknochens einzuschlagen.

Ferner ist es vorteilhaft, wenn das Instrumentarium ein Einschlagwerkzeug aufweist. Mit diesem kann der Raspelschaft einfach und sicher in die Knochenkavität des Femurs eingeschlagen werden.

Die eingangs gestellte Aufgabe wird ferner bei einem Implantationsset der eingangs beschriebenen Art erfindungsgemäß dadurch gelöst, dass die durch die mindestens zwei Tiefenanschläge definierten Tiefenanschlagflächen unterschiedliche Oberflächenstrukturen aufweisen oder unterschiedlich eingefärbt sind, dass die mindestens zwei unterschiedlichen Implantatteile jeweils ein Kennzeichnungselement umfassen, dass sich die Kennzeichnungselemente der mindestens zwei unterschiedlichen Implantatteile voneinander unterscheiden und dass jedem Kennzeichnungselement der mindestens zwei unterschiedlichen Implantatteile einer der mindestens zwei Tiefenanschläge zugeordnet ist.

Mit dem erfindungsgemäß vorgeschlagenen Implantationsset ist es insbesondere möglich, mit nur einem Raspelinstrument eine Knochenkavität eines Femurknochens für die Implantation unterschiedlicher Implantatteile vorzubereiten, insbesondere für die Implantation unterschiedlicher Prothesenschäfte zur Behandlung unterschiedlicher Indikation. Die Handhabung des Instrumentariums wird weiter vereinfacht dadurch, dass jeder der mindestens zwei Tiefenanschläge mindestens eine Tiefenanschlagfläche umfasst. Ein Tiefenanschlag kann eine, zwei oder mehr Tiefenanschlagflächen umfassen. Diese kennzeichnen vorzugweise dieselbe Einschlagtiefe für den Raspelschaft. Günstig ist es, dass die durch die mindestens zwei Tiefenanschläge definierten Tiefenanschlagflächen unterschiedliche Oberflächenstrukturen aufweisen oder unterschiedlich eingefärbt sind. Insbesondere ermöglichen Farben einem Operateur auf einfache Weise eine Unterscheidung der mindestens zwei Tiefenanschlagflächen. So kann er sicher erkennen, wie weit der Raspelschaft bereits in den Femurknochen eingetrieben ist und ob er gegebenenfalls noch weiter eingetrieben werden muss. Ferner ist es günstig, dass die mindestens zwei unterschiedlichen Implantatteile jeweils ein Kennzeichnungselement umfassen und wenn sich die Kennzeichnungselemente der mindestens zwei unterschiedlichen Implantatteile voneinander unterscheiden. Die Kennzeichnungselemente erlauben es einem Operateur und seinem Hilfspersonal, sicher zwischen unterschiedlichen Implantatteilen, beispielsweise Prothesenschäften zur Behandlung von Coxa vara, Coxa norma und Coxa valgus, gezielt und richtig aus einem bereitgestellten Satz von Implantatteilen, die das Implantatset bilden, auszuwählen. Besonders sicher lassen sich vom Operateur ausgewählte Implantatteile implantieren, da jedem Kennzeichnungselement der mindestens zwei unterschiedlichen Implantatteile einer der mindestens zwei Tiefenanschläge zugeordnet ist. Wählt ein Operateur beispielsweise ein Implantatteil mit einem individuell ausgebildetem Kennzeichnungselement zur Implantation aus, dann weiß er sofort, welcher der mindestens zwei Tiefenanschläge am Raspelschaft zum ausgewählten Implantatteil korrespondiert und damit auch wie weit er den Raspelschaft in den Knochen einschlagen muss.

Vorzugsweise unterscheiden sich die Kennzeichnungselemente der mindestens zwei unterschiedlichen Implantatteile farblich, aufgrund ihrer Oberflächenstruktur und/oder durch ihre Position am mindestens einen Implantatteil voneinander. So kann ein Operateur die unterschiedlichen Implantatteile auf einfache Weise unterscheiden, beispielsweise aufgrund ihrer jeweiligen farblichen Gestaltung oder ihrer Oberflächenstruktur oder in Abhängigkeit eines Ortes oder einer Position, an welcher sie am Implantatteil angeordnet oder ausgebildet sind.

Besonders einfach und sicher lassen sich Implantatteile und Tiefenanschläge einander zuordnen, wenn eine Einfärbung der mindestens zwei Tiefenanschläge einer Einfärbung der Kennzeichnungselemente der mindestens zwei unterschiedlichen Implantatteile entspricht. Mit anderen Worten gehören gleiche Farben zusammen. Wird ein Implantatteil vom Operateur ausgewählt, kann er anhand der Farbe des Kennzeichnungselements sofort erkennen, welcher Tiefenanschlag am Raspelschaft zum ausgewählten Implantatteil korrespondiert. Damit weiß der Operateur auch, wie weit er den Raspelschaft in den Knochen eintreiben muss.

Insbesondere ist es günstig, wenn die Kennzeichnungselemente der mindestens zwei unterschiedlichen Implantatteile mit denselben Kennzeichnungsfarben der Tiefenanschläge einfärbt sind. So lässt sich der bei der Vorbereitung der Femurkavität zu berücksichtigende Tiefenanschlag einfach und sicher bestimmen, wenn die Kennzeichnungsfarbe des Kennzeichnungselements vom ausgewählten Implantatteil bekannt ist.

Auf besonders einfache Weise ausbilden lassen sich die Kennzeichnungselemente der mindestens zwei unterschiedlichen Implantatteile, wenn sie in Form geometrischer Flächenbereiche ausgebildet sind. Beispielsweise können auf einer äußeren Oberfläche des Implantatteils entsprechende geometrische Flächenbereiche farblich oder durch eine leicht erkennbare Oberflächenstruktur ausgebildet werden.

Auf besonders einfache Weise ausbilden lassen sich die Kennzeichnungselemente, wenn die geometrischen Flächenbereiche in Form von Kreisflächen oder Vielecken ausgebildet sind. Diese lassen sich beispielsweise einstückig mit den Implantatteilen ausbilden oder in Form von Klebeetiketten aufbringen.

Besonders gut und schnell erkennen kann ein Operateur die Art des von ihm ausgewählten Implantatteils, wenn die geometrischen Flächenbereiche an in proximaler Richtung weisenden Halsendflächen von Hälsen der Implantatteile angeordnet oder ausgebildet sind. Diese weisen auch nach der Implantation in seine Richtung, sodass er abschließend nochmals prüfen kann, ob das korrekte Implantatteil eingesetzt wurde.

Vorteilhaft ist es, wenn die Kennzeichnungselemente mit den mindestens zwei unterschiedlichen Implantatteilen lösbar verbindbar ausgebildet oder unlösbar angeordnet oder ausbildet sind. Insbesondere lösbar verbindbare Kennzeichnungselemente haben den Vorteil, dass sie auf einfache Weise während oder nach dem chirurgischen Eingriff entfernt werden können. Beispielweise können sie zur Dokumentationszwecken aufbewahrt werden, um die Art des eingesetzten Implantatteils zu dokumentieren.

Vorzugsweise sind die Kennzeichnungselemente in Form von Schutzkappen ausgebildet. Beispielsweise können diese ausgebildet sein, um auf Hälse von Implantatschäften aufgesteckt oder aufgeclipst zu werden. Sie dienen dann einem Operateur insbesondere zur Identifikation des Implantatteils und optional auch zum Schutz desselben. Insbesondere eine Beschädigung des Halses des Hüftgelenkschafts birgt die Gefahr, dass keine sichere Verbindung mit dem Kugelgelenkkopf mehr erreicht werden kann.

Ferner ist es günstig, wenn die Kennzeichnungselemente mit den mindestens zwei unterschiedlichen Implantatteilen kraft- und/oder formschlüssig verbindbar sind. So kann insbesondere sichergestellt werden, dass sich die Kennzeichnungselemente nicht in unerwünschter Weise von den mit ihnen gekennzeichneten Implantatteilen lösen können.

Die nachfolgende Beschreibung bevorzugter Ausführungsformen der Erfindung dient im Zusammenhang mit den Zeichnungen der näheren Erläuterung. Es zeigen:
- Figur 1a:: eine schematische Darstellung einer Coxa valga;
- Figur 1b:: eine schematische Darstellung einer Coxa norma;
- Figur 1c:: eine schematische Darstellung einer Coxa vara;
- Figur 2a:: eine schematische Darstellung übereinanderliegender Konturen dreier Hüftschäfte für die Indikationen Coxa valga, Coxa norma und Coxa vara;
- Figur 2b:: eine Ansicht des Hüftschafts für die Indikation Coxa valga aus Figur 2a in Richtung des Pfeils A;

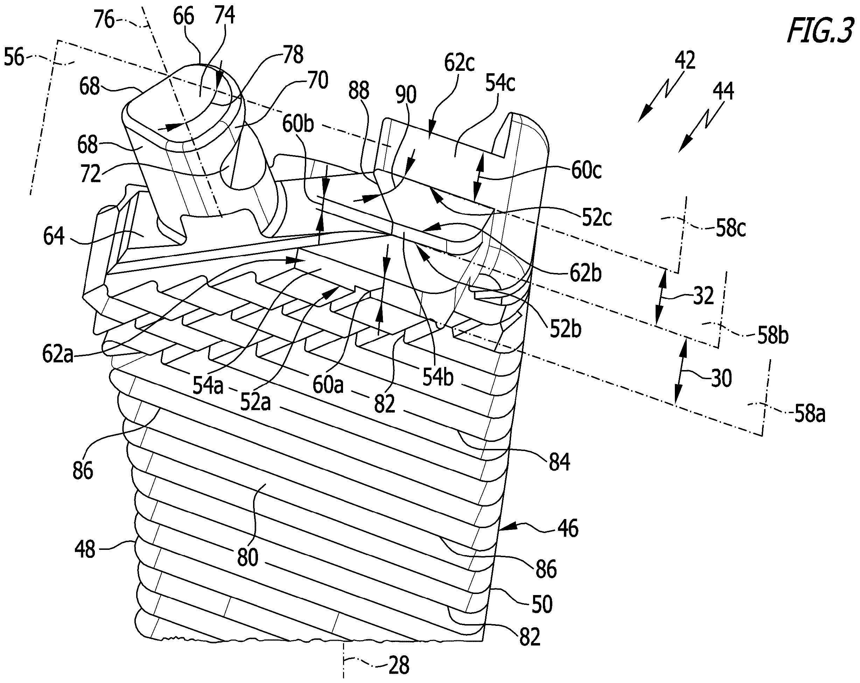

- Figur 3:: eine schematische perspektivische Teilansicht eines nicht erfindungsgemäßen Raspelschafts mit drei Tiefenanschlägen;
- Figur 4:: eine schematische perspektivische Teilansicht eines weiteren Ausführungsbeispiels eines Raspelschafts mit drei Tiefenanschlägen;
- Figur 5a:: eine schematische Teilansicht eines Hüftschafts für die Indikation Coxa vara;
- Figur 5b:: eine schematische Teilansicht eines Hüftschafts für die Indikation Coxa norma;
- Figur 5c:: eine schematische Teilansicht eines Hüftschafts für die Indikation Coxa valga; und
- Figur 6:: eine schematische Darstellung von Teilansichten weiterer Ausführungsbeispiele von Hüftschäften für die Indikationen Coxa valga, Coxa norma und Coxa vara mit dem in Figur 4 teilweise dargestellten Raspelschaft.

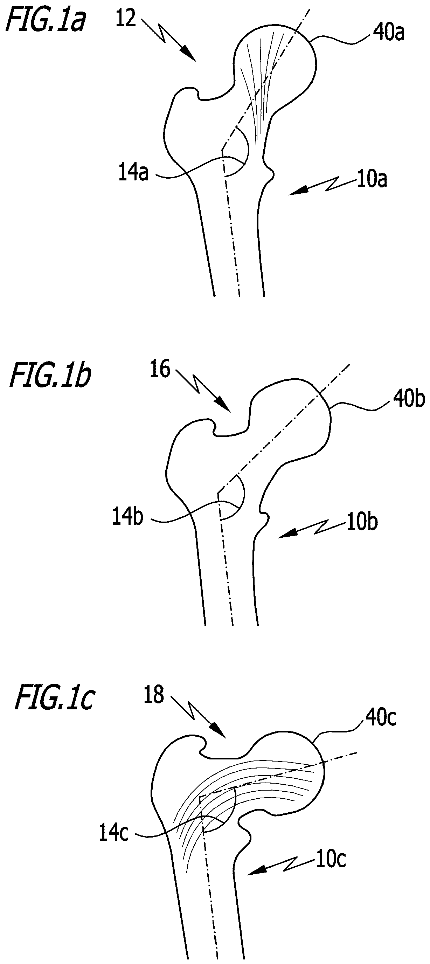

In Figur 1a ist ein Femurknochen 10a einer Coxa valga 12 schematisch dargestellt mit einem CCD-Winkel 14a, der größer als 140° ist.

In Figur 1b ist ein Femurknochen 10b einer Coxa norma 16 schematisch dargestellt mit einem CCD-Winkel 14b von etwa 125°.

In Figur 1c ist ein weiterer Femurknochen 10c einer Coxa vara 18 schematisch dargestellt mit einem CCD-Winkel 14c, der kleiner als 120° ist.

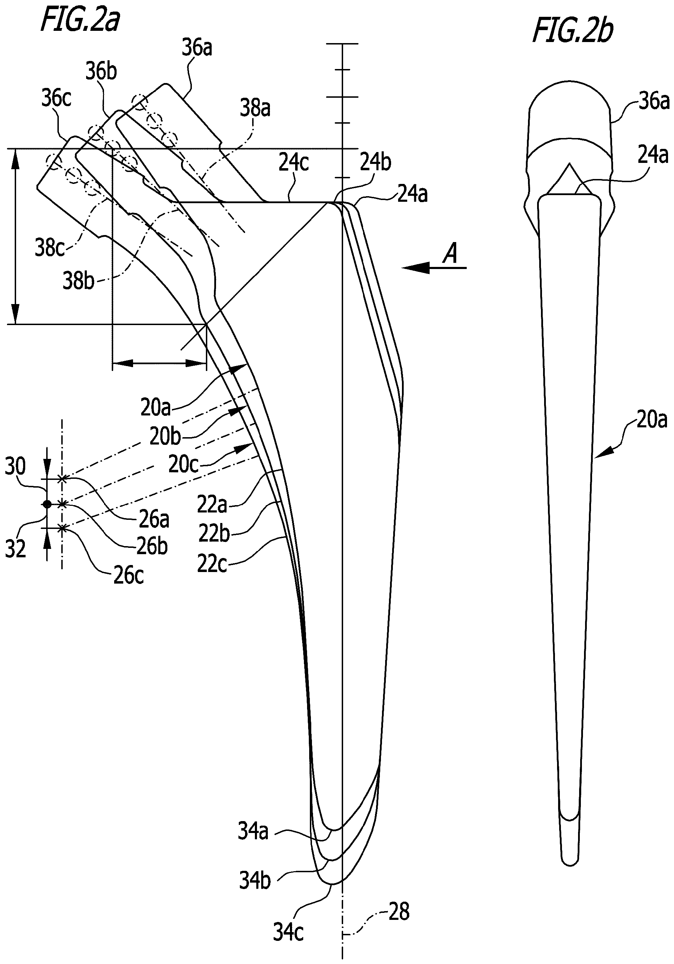

Zur Behandlung der drei in den Figuren 1a bis 1c schematisch dargestellten pathologischen Situationen dienen die drei in Figur 2a schematisch dargestellten Konturen der Prothesenschäfte 20a, 20b und 20c. Mediale Seitenflächen 22a, 22b und 22c der Prothesenschäfte 20a, 20b und 20c sind von diesen weg weisend konkav gekrümmt und weisen alle den identischen Krümmungsradius auf. Werden proximale Endflächen 24 der Prothesenschäfte 20a, 20b und 20c bündig zueinander ausgerichtet, wie beispielhaft für deren Konturen in Figur 2a dargestellt, so hat dies zu Folge, dass Mittelpunkte 26a, 26b und 26c von an die Seitenflächen 22a, 22b und 22c anliegenden Berührkreisen bezogen auf eine durch die Prothesenschäfte 20a, 20b und 20c definierte Längsrichtung 28 relativ zueinander versetzt sind, und zwar um einen Abstand 30 zwischen den Mittelpunkten 26a und 26b und einem Abstand 32 zwischen den Mittelpunkten 26b und 26c.

Wie in Figur 2a gut zu erkennen, ist eine Gesamtlänge des Prothesenschafts 20c größer als eine Gesamtlänge des Prothesenschafts 20b, welcher wiederum länger ist als eine Gesamtlänge des Prothesenschafts 20a. Distale Enden 34a, 34b und 34c der Prothesenschäfte 20a, 20b und 20c sind umso weiter von den jeweiligen Endflächen 24a, 24b und 24c entfernt, je kleiner der CCD-Winkel 14a, 14b beziehungsweise 14c ist.

Hälse 36a, 36b und 36c der Prothesenschäfte 20a, 20b und 20c definieren Halslängsachsen 38a, 38b und 38c, die mit der Längsrichtung 28 jeweils einen Winkel einschließen, welcher umso größer ist, je kleiner der CCD-Winkel 14a, 14b und 14c ist.

Für die Implantation der Prothesenschäfte 20a, 20b und 20c müssen die Femurknochen 10a, 10b und 10c teilreseziert werden durch Abtrennen der Femurköpfe 40a, 40b und 40c. Ein Markkanal der Femurknochen 10a, 10b und 10c wird dann mit einem medizinischen Instrumentarium 42 umfassend ein Raspelinstrument 44 präpariert. Das Raspelinstrument 44 umfasst einen Raspelschaft 46 mit einer medialen Seitenfläche 48, die einen Krümmungsradius aufweist, welcher dem Krümmungsradius der Seitenflächen 22a, 22b und 22c der Prothesenschäfte 20a, 20b und 20c entspricht. Eine laterale Seitenfläche 50 des Raspelschafts 46 verläuft nahezu parallel zur Längsrichtung 28.

Damit der gleiche Raspelschaft 46 zur Präparation der Femurknochen 10a, 10b und 10c genutzt werden kann, muss er zur Vorbereitung des Markraums des jeweiligen Femurknochen 10a, 10b 10c unterschiedlich tief eingeschlagen werden, und zwar umso tiefer je kleiner der CCD-Winkel 14a, 14b beziehungsweise 14c der jeweiligen Indikation ist. Mit anderen Worten muss der Raspelschaft 46 um den Abstand 32 tiefer eingeschlagen werden für die Implantation des Prothesenschafts 20c für die Indikation Coxa vara im Vergleich zur Indikation Coxa norma. Dagegen muss der Raspelschaft 46 um den Abstand 30 tiefer in den Femurknochen 10b tiefer eingeschlagen werdenbei der Indikation Coxa norma im Vergleich zur Indikation Coxa valga 12.

Damit ein Operateur den Raspelschaft 46 entsprechend tief in den jeweiligen Femurknochen 10a, 10b und 10c einschlagen kann, sind am Raspelschaft drei Tiefenanschläge 52a, 52b und 52c ausgebildet. Der Tiefenanschlag 52a umfasst zwei Tiefenanschlagflächen 54a, der Tiefenanschlag 52b umfasst zwei Tiefenanschlagflächen 54b. Der Tiefenanschlag 52c umfasst eine einzige Tiefenanschlagfläche 54c. Insgesamt ist Raspelschaft 46 spiegelsymmetrisch zu einer die Längsrichtung 28 enthaltenden Symmetrieebene 56 ausgebildet.

Die Tiefenanschlagflächen 54a, 54b und 54c definieren Tiefenanschlagflächenebenen 58a, 58b und 58c, die parallel zueinander verlaufen. Ein Abstand zwischen der Tiefenanschlagflächenebene 58a und 58b entspricht dem Abstand 30, ein Abstand zwischen der Tiefenanschlagflächenebene 58b und 58c entspricht dem Abstand 32. Bei den in den Figuren schematisch dargestellten Prothesenschäften 20a, 20b und 20c entspricht der Abstand 30 dem Abstand 32. Mit anderen Worten sind die Tiefenanschlagflächen 54a, 54b und 54c äquidistant zueinander ausgebildet.

Die Tiefenanschlagflächen 54a, 54b und 54c bilden einen Teil von in proximaler Richtung weisenden Endflächenbereichen 62a, 62b und 62c. Eine Breite der Tiefenanschlagfläche 54c ist größer als eine Breite der beiden Tiefenanschlagflächen 54a, welche wiederum doppelt so breit sind wie die Tiefenanschlagflächen 54b.

Die proximalste Tiefenanschlagfläche 54c korrespondiert zur Indikation Coxa vara 18, die Tiefenanschlagfläche 54b zur Indikation Coxa norma 16 und die Tiefenanschlagfläche 54a zur Indikation Coxa valga 12.

Eine gegenüber den Tiefenanschlagflächen 54b nach medial geneigte Kopplungsendfläche 64 des Raspelschafts 46 trägt ein symmetrisch zur Symmetrieebene ausgebildetes Kopplungselement 66 zum Koppeln des Raspelschafts 46 mit einem in den Figuren nicht dargestellten Griffelement. Dieses kann insbesondere eine Einschlagfläche aufweisen, um den mit dem Griffelement gehaltenen Raspelschaft 46 in die Kavitäten der Femurknochen 10a, 10b und 10c einzuschlagen.

Das Kopplungselement 66 weist zwei gegeneiner geneigte und in medialer Richtung weisende Seitenflächen 68 auf, welche über eine in lateraler Richtung weisende Zylinderfläche 70 miteinander verbunden sind. Im Bereich der Zylinderfläche ist in lateraler Richtung weisend eine Ausnehmung 72 ausgebildet. Eine in proximaler Richtung weisende Endfläche 74 des Kopplungselements 66 liegt unterhalb der Tiefenanschlagflächenebene 58c, also vollständig distal derselben. Eine Kopplungselementlängsachse 76 des Kopplungselements 66 ist gegenüber den Tiefenanschlagflächenebenen 58a, 58b und 58c um einen Winkel 78 geneigt, welcher kleiner ist als 90°. Vorzugweise liegt er in einem Bereich von 50° bis 80°. Die Kopplungselementlängsachse 76 liegt zudem in der Symmetrieebene 56.

Der Raspelschaft 46 umfasst ferner zwei voneinander weg weisende Seitenflächen 80, die die Seitenflächen 48 und 50 miteinander verbinden. Die Seitenflächen 80 verlaufen im Wesentlichen parallel zueinander und sind spiegelsymmetrisch zur Symmetrieebene 56 ausgebildet. Die beiden Seitenflächen 80 sowie die Seitenflächen 48 und 50 weisen eine Vielzahl von Nuten 82 auf, die parallel zueinander verlaufen und scharfe Kanten 84 aufweisen, die Raspelzähne 86 bilden.

Die distalsten Tiefenanschlagflächen 54a grenzen an die Seitenflächen 80 an und verlaufen quer zu diesen, vorzugsweise senkrecht.

Die Seitenfläche 50 erstreckt sich in proximaler Richtung bis im Wesentlichen auf die Höhe der Tiefenanschlagfläche 54c. Ferner sind die Tiefenanschlagflächen 54b und 54a jeweils etwas in medialer Richtung bezogen auf die Seitenfläche 50 versetzt.

Die Tiefenanschlagfläche 54c ist an einem im Wesentlichen quaderförmigen Vorsprung ausgebildet, welcher eine in medialer Richtung weisende Stirnfläche 88 aufweist, welche im Wesentlichen parallel zur Kopplungselementlängsachse 76 verläuft. Die Stirnfläche 88 schließt mit der Tiefenanschlagfläche 54c einen Winkel 90 ein, welcher einen Wert in einem Bereich von etwa 50° bis 70°, vorzugsweise 60°, aufweist. Somit erstrecken sich die Tiefenanschlagflächen 54a am weitesten in medialer Richtung. Ferner steht die Tiefenanschlagfläche 54c in medialer Richtung über die Tiefenanschlagflächen 54b vor.

Die drei Tiefenanschläge 52a, 52b und 52c ermöglichen es, dass das Raspelinstrument 44 zur Vorbereitung für alle Femurknochen 10a, 10b und 10c genutzt werden kann, unabhängig davon, ob der Prothesenschaft 20a, 20b und 20c implantiert werden soll. Der Raspelschaft 46 wird abhängig von dem zu implantierenden Prothesenschaft 20a, 20b und 20c nur so weit in den teilweise resezierten Femurknochen eingeschlagen, bis die der jeweiligen Indikation zugeordnete Tiefenanschlagfläche 54a, 54b und 54c auf derselben Höhe liegt wie einen Resektionsebene der Femurknochen 10a, 10b und 10c. Durch das Vorsehen der Tiefenanschläge 52a, 52b und 52c am Raspelschaft 46 kann auf die Bereitstellung von zwei weiteren Raspelschäften für die beiden anderen Indikationen verzichtet werden.

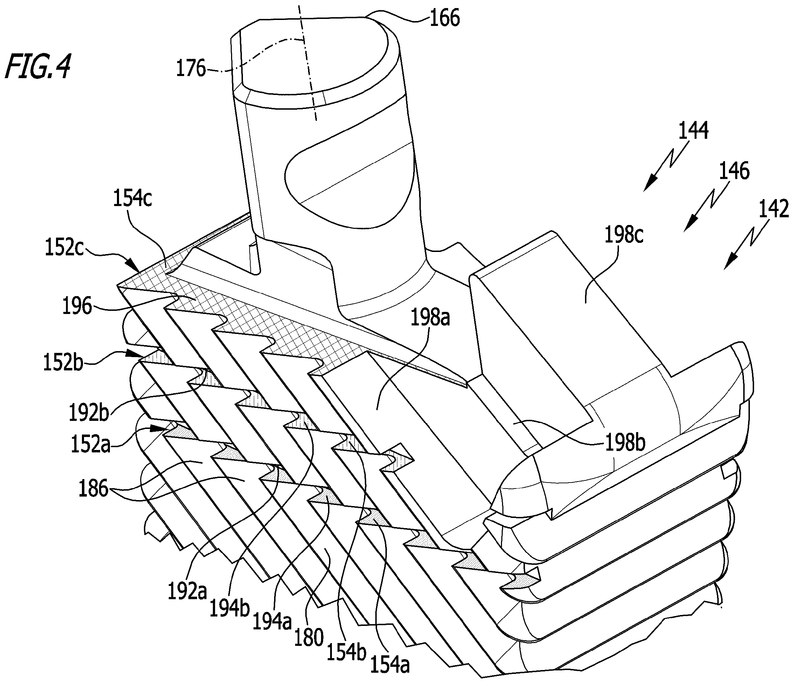

In Figur 4 ist ein weiteres Ausführungsbeispiel eines insgesamt mit dem Bezugszeichen 144 bezeichneten Raspelinstruments teilweise dargestellt. Es stimmt in seinem grundsätzlichen Aufbau mit dem Raspelinstrument 44 überein, sodass identische Elemente mit denselben Bezugszeichen bezeichnet sind, wobei beim Raspelinstrument 144 jeweils noch die Ziffer "1" vorangestellt ist.

Das Raspelinstrument 144 umfasst einen Raspelschaft 146, welcher insgesamt drei Tiefenanschläge 152a, 152b und 152c aufweist. Die Tiefenanschläge 152a und 152b sind in Form von Nuten 192a und 192b in den Seitenflächen 180 ausgebildet, und zwar quer zu den Raspelzähnen 186 verlaufend. In proximaler Richtung, also in Richtung auf das Kopplungselement 166 hin weisende Nutseitenflächen 194a und 194b definieren Tiefenanschlagflächen 154a und 154b. Eine Endfläche 196 des Raspelschafts, von welchem sich das Kopplungselement 166 in proximaler Richtung weg erstreckt, definiert die Tiefenanschlagfläche 154c des Tiefenanschlags 152c.

Die Tiefenanschlagflächen 154a, 154b und 154c definieren Tiefenanschlagflächenebenen 158a, 158b und 158c, die parallel zueinander verlaufen. Ein Abstand 130 zwischen der Tiefenanschlagflächenebene 158a und der Tiefenanschlagflächenebene 158b entspricht dem Abstand 132 zwischen der Tiefenanschlagflächenebene 158b und der Tiefenanschlagflächenebene 158c. Somit sind die beiden Nuten 192a und 192b sowohl von der Endfläche 196 als auch voneinander beabstandet.

Ferner verlaufen die Tiefenanschlagflächenebenen 158a, 158b und 158c quer zur vom Raspelschaft 146 definierten Längsrichtung 128.

Der Tiefenanschlag 152a korrespondiert zur Indikation Coxa valga und bildet somit eine entsprechende Einschlagmarkierung für einen Operateur.

Der Tiefenanschlag 152b korrespondiert zur Indikation Coxa norma und bildet eine hierfür entsprechende Einschlagmarkierung.

Ferner definiert der Tiefenanschlag 152c eine Einschlagmarkierung für die Indikation Coxa vara.

Die beim Raspelschaft 46 als Tiefenanschlagflächen 54a, 54b und 54c bezeichneten Flächen des Raspelschafts 146 definieren Schulterhöhenflächen 198a, 198b und 198c. Die Schulterhöhenfläche 198a korrespondiert zur Schulterhöhe des Prothesenschafts 120a, die Schulterhöhenfläche 198b korrespondiert zur Schulterhöhe des Prothesenschafts 120b und die Schulterhöhenfläche 198c korrespondiert zur Schulterhöhe des Prothesenschafts 120c.

Um die zum jeweiligen Tiefenanschlag 152a, 152b und 152c korrespondierende Indikation für einen Operateur leicht erkennbar zu machen, sind die Tiefenanschlagflächen 154a, 154b und 154c unterschiedlich eingefärbt. Dies ist in Figur 4 schematisch durch unterschiedliche Schraffuren dargestellt. Optional können die Tiefenanschlagflächen 154a, 154b und 154c auch mit unterschiedlichen Oberflächenstrukturen versehen sein, die eine leichte Erkennbarkeit der jeweiligen Tiefenanschläge 152a, 152b und 152c für einen Operateur gewährleisten.

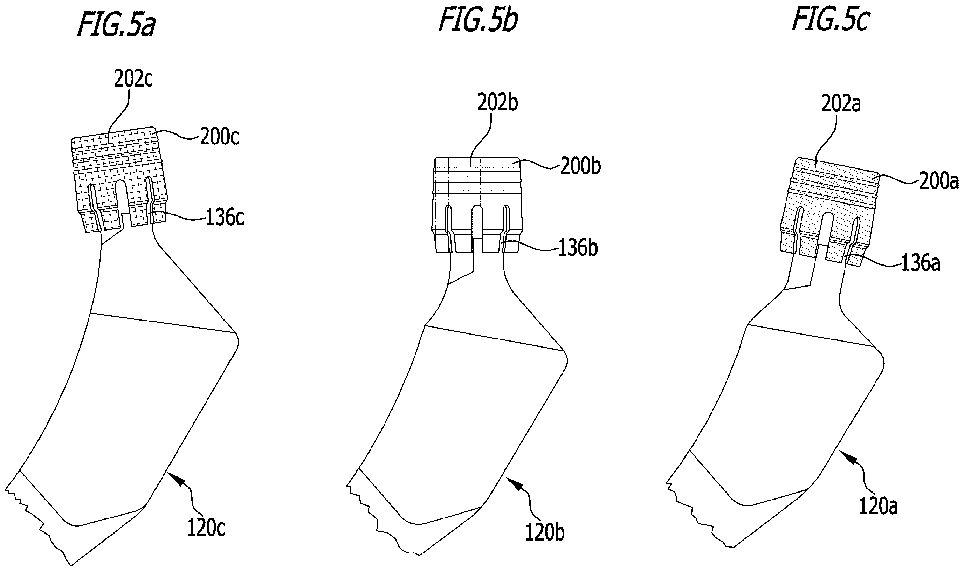

Um einem Operateur die Präparation eines Femurknochens zu erleichtern, sind optional, wie in den Figuren 5a bis 5c beispielhaft dargestellt, Kennzeichnungselemente 200a, 200b und 200c in Form von Schutzkappen 202a, 202b und 202c ausgebildet, die auf die Hälse 136a, 136b und 136c der Prothesenschäfte 120a, 120b und 120c aufbringbar beziehungsweise temporär mit diesen koppelbar sind.

Die Kennzeichnungselemente 200a, 200b und 200c sind optional entsprechend der Indikation des jeweiligen Prothesenschafts 120a, 120b und 120c eingefärbt, und zwar mit denselben Farben, die zur Kennzeichnung der Tiefenanschläge 152a, 152b und 152c dienen, also beispielsweise blau für die Indikation Coxa vara, orange für die Indikation Coxa norma und grün für die Indikation Coxa valga. So kann beispielsweise ein Operateur den zur Indikation Coxa norma korrespondierenden Schaft 120b wählen und weiß dann sofort, dass er den Raspelschaft 146 bis zum Tiefenanschlag 152b, dessen Tiefenanschlagfläche 154b mit derselben Farbe wie das Kennzeichnungselement 200b eingefärbt ist, in den Femurknochen einschlagen muss, um diesen für die Implantation des Prothesenschafts 120b optimal zu präparieren.

Die Schutzkappen 202a, 202b und 202c können optional eine Kopplungseinrichtung aufweisen, welche eine kraft- und/oder formschlüssige Verbindung mit den Hälsen 136a, 136b und 136c ermöglicht. Die Schutzkappen 202a, 202b und 202c schützen zum einen die Hälse 136a, 136b und 136c vor Beschädigungen und dienen zum anderen zur Kennzeichnung der Indikation des jeweiligen Prothesenschafts 120a, 120b und 120c.

Nach dem Einsetzen der Prothesenschäfte 120a, 120b und 120c werden die Schutzkappen 202a, 202b und 202c entfernt und ein für den Patienten passender Gelenkkopf auf die Hälse 136a, 136b und 136c aufgesetzt.

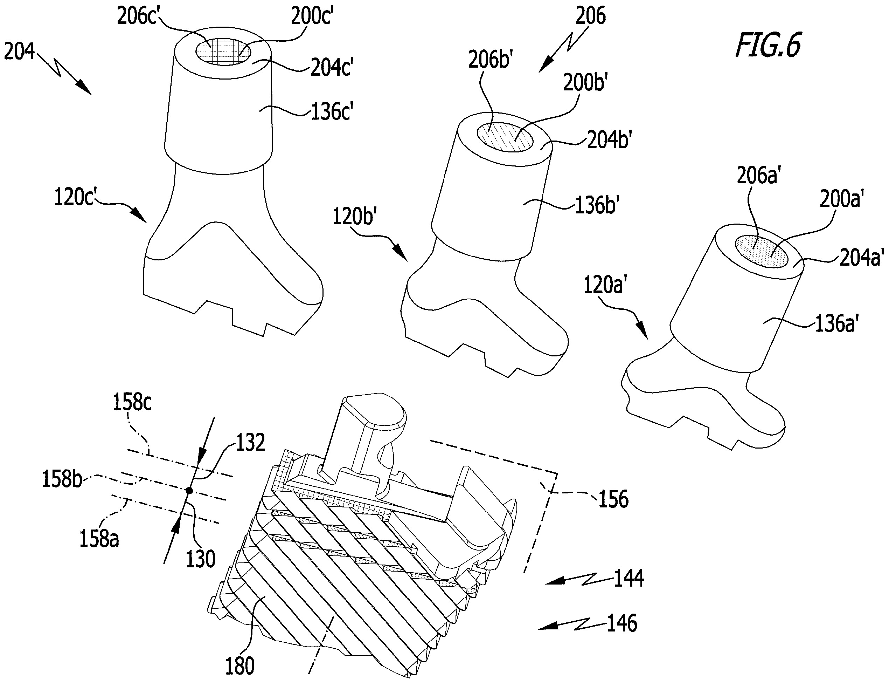

In Figur 6 ist beispielhaft ein Implantationsset 204 schematisch dargestellt umfassend insgesamt drei unterschiedliche Implantatteile in Form von Prothesenschäften 120a', 120b' und 120c'.

Die Prothesenschäfte 120a', 120b' und 120c' unterscheiden sich von den Prothesenschäften 120a, 120b und 120c dadurch, dass sie andere Kennzeichnungselemente 200a', 200b' und 200c' umfassen. Die Kennzeichnungselemente 200a', 200b' und 200c' sind auf in proximaler Richtung weisenden ebenen Endflächen 204a', 204b' und 204c' der Hälse 136a', 136b' und 136c' angeordnet. Sie sind in Form geometrischer Flächenbereiche 206a', 206b' und 206c' ausgebildet, die jeweils kreisförmig sind.

Die Flächenbereiche 206a', 206b' und 206c' sind wiederrum eingefärbt, und zwar mit denselben Farben wie die Tiefenanschlagflächen 154a, 154b und 154c des Raspelschafts 146.

Wählt der Operateur beispielsweise den Prothesenschaft 120a' zur Behandlung der Indikation Coxa valga, so muss er den Raspelschaft 146 nur bis zum Tiefenanschlag 152a in den Femurknochen einschlagen.

Die drei Prothesenschäfte 120a', 120b' und 120c' bilden ein modulares Implantatset 206 und zusammen mit dem Raspelinstrument 144 das Implantationsset 204.

### Bezugszeichenliste

- 10a, 10b, 10c: Femurknochen
- 12: Coxa valga
- 14a, 14b, 14c: CCD-Winkel
- 16: Coxa norma
- 18: Coxa vara
- 20a, 20b, 20c: Prothesenschaft
- 22a, 22b, 22c: Seitenflächen
- 24: Endflächen
- 26a, 26b, 26c: Mittelpunkte
- 28: Längsrichtung
- 30: Abstand
- 32: Abstand
- 34: Ende
- 36a, 36b, 36c: Hals
- 38: Halslängsachse
- 40a, 40b, 40c: Femurkopf
- 42: Instrumentarium
- 44: Raspelinstrument
- 46: Raspelschaft
- 48: Seitenflächen
- 50: Seitenflächen
- 52a, 52b, 52c: Tiefenanschlag
- 54a, 54b, 54c: Tiefenanschlagfläche
- 56: Symmetrieebene
- 58a, 58b, 58c: Tiefenanschlagflächenebene
- 60a, 60b, 60c: Breite
- 62a, 62b, 62c: Endflächenbereiche
- 64: Kopplungsendfläche
- 66: Kopplungselement
- 68: Seitenflächen
- 70: Zylinderfläche
- 72: Ausnehmung
- 74: Endfläche
- 76: Kopplungselementlängsachse
- 78: Winkel
- 80: Seitenflächen
- 82: Nut
- 84: Kante
- 86: Raspelzahn
- 88: Stirnfläche
- 90: Winkel
- 120a, 120b, 120c: Prothesenschaft
- 128: Längsrichtung
- 130: Abstand
- 132: Abstand
- 136a, 136b, 136c: Hals
- 142: Instrumentarium
- 144: Raspelinstrument
- 146: Raspelschaft
- 152a, 152b, 152c: Tiefenanschlag
- 154a, 154b, 154c: Tiefenanschlagfläche
- 156: Symmetrieebene
- 158a, 158b, 158c: Tiefenanschlagflächenebene
- 166: Kopplungselement
- 176: Kopplungselementlängsachse
- 180: Seitenfläche
- 186: Raspelzahn
- 192a, 192b: Nut
- 194a, 194b: Nutseitenflächen
- 196: Endfläche
- 198a, 198b, 198c: Schulterhöhenflächen
- 200a, 200b, 200c: Kennzeichnungselement
- 202a, 202b, 202c: Schutzkappe
- 204: Implantationsset
- 206: Implantatset
- 120a', 120b', 120c': Prothesenschaft
- 200a', 200b', 200c': Kennzeichnungselement
- 204a', 204b', 204c': Endfläche
- 206a', 206b', 206c': Flächenbereich

## Patentansprüche

1. Medizinisches Instrumentarium (42; 142), insbesondere zum Implantieren eines Hüftgelenkschafts (20a, 20b, 20c; 120a, 120b, 120c; 120a', 120b', 120c'), umfassend ein Raspelinstrument (44; 144) mit einem eine Längsrichtung (28; 128) definierenden Raspelschaft (46; 146), wobei am Raspelschaft (46; 146) mindestens zwei Tiefenanschläge (152a, 152b, 152c) angeordnet oder ausgebildet sind, wobei mindestens zwei der mindestens zwei Tiefenanschläge (152a, 152b) mindestens eine Nut (192a, 192b) umfassen, **dadurch gekennzeichnet, dass** die Nuten (192a, 192b) am Raspelschaft (146) parallel oder im Wesentlichen parallel zu einer in proximaler Richtung weisenden Endfläche (196) des Raspelschafts (146) verlaufen, dass das Instrumentarium ein am Raspelschaft (146) angeordnetes oder ausgebildetes Kopplungselement (166) zum Koppeln des Raspelschafts (146) mit einem Griffelement umfasst, dass das Kopplungselement (166) von der Endfläche (196) in proximaler Richtung oder im Wesentlichen in proximaler Richtung weisend abstehend angeordnet oder ausgebildet ist und dass die durch die mindestens zwei Tiefenanschläge (152a, 152b, 152c) definierten Tiefenanschlagflächen (154a, 154b, 154c) unterschiedliche Oberflächenstrukturen aufweisen oder unterschiedlich eingefärbt sind.

2. Medizinisches Instrumentarium nach Anspruch 1, **dadurch gekennzeichnet, dass** jeder der mindestens zwei Tiefenanschläge (152a, 152b, 152c) mindestens eine Tiefenanschlagfläche (154a, 154b, 154c) umfasst.

3. Medizinisches Instrumentarium nach Anspruch 2, **dadurch gekennzeichnet, dass** die in proximaler Richtung weisende Endfläche (196) des Raspelschafts (146) die mindestens eine Tiefenanschlagfläche (154c) umfasst.

4. Medizinisches Instrumentarium nach einem der voranstehenden Ansprüche, **dadurch gekennzeichnet, dass** die mindestens zwei Tiefenanschläge (152a, 152b, 152c) voneinander beabstandet angeordnet oder ausgebildet sind.

5. Medizinisches Instrumentarium nach einem der voranstehenden Ansprüche, **dadurch gekennzeichnet, dass** mindestens zwei der mindestens zwei Tiefenanschläge (152a, 152b) mindestens eine Nut (192a, 192b) umfassen.

6. Medizinisches Instrumentarium nach einem der voranstehenden Ansprüche, **dadurch gekennzeichnet, dass**
a) die mindestens eine Nut (192a, 192b) eine Nutlängsrichtung definiert und/oder geradlinig oder im Wesentlichen geradlinig verläuft
und/oder
b) die mindestens eine Nut (192a, 192b) auf einer Seitenfläche (180) des Raspelschafts (146) ausgebildet ist, welche in einer Richtung quer zur Längsrichtung weist.

7. Medizinisches Instrumentarium nach Anspruch 2 oder einem der Ansprüche 3 bis 6, **dadurch gekennzeichnet, dass** die mindestens eine Nut (192a, 192b) mindestens eine Nutseitenfläche (194a, 194b) umfasst und dass die mindestens eine Nutseitenfläche (194a, 194b) die mindestens eine Tiefenanschlagfläche (154a, 154b) definiert.

8. Medizinisches Instrumentarium nach Anspruch 2 oder einem der Ansprüche 3 bis 7, soweit diese direkt oder indirekt auf Anspruch 2 rückbezogen sind, **dadurch gekennzeichnet, dass** die mindestens eine Tiefenanschlagfläche (154a, 154b, 154c) eben ist.

9. Medizinisches Instrumentarium nach Anspruch 2 oder einem der Ansprüche 3 bis 8, soweit diese direkt oder indirekt auf Anspruch 2 rückbezogen sind, **dadurch gekennzeichnet, dass** die mindestens eine Tiefenanschlagfläche (154a, 154b, 154c) eines der mindestens zwei Tiefenanschläge (152a, 152b, 152c) parallel oder im Wesentlichen parallel zu der mindestens einen Tiefenanschlagfläche (154a, 154b, 154c) eines anderen der mindestens zwei Tiefenanschläge (152a, 152b, 152c) verläuft.

10. Medizinisches Instrumentarium nach Anspruch 2 oder einem der Ansprüche 3 bis 9, soweit diese direkt oder indirekt auf Anspruch 2 rückbezogen sind, **dadurch gekennzeichnet, dass** mindestens zwei Tiefenanschlagflächen (154a, 154b, 154c) Tiefenanschlagflächenebenen (158a, 158b, 158c) definieren, welche quer, insbesondere senkrecht, zur Längsrichtung (128) verlaufen.

11. Medizinisches Instrumentarium nach einem der Ansprüche 2 bis 10, **dadurch gekennzeichnet, dass**
a) der Raspelschaft (146) symmetrisch oder im Wesentlichen symmetrisch zu einer Symmetrieebene (56) ausgebildet ist, welche quer, insbesondere senkrecht, zu mindestens einer der Tiefenanschlagflächen (154a, 154b, 154c) verläuft,
und/oder
b) dass drei Tiefenanschlagflächen (154a, 154b, 154c) vorgesehen sind, die äquidistant oder im Wesentlichen äquidistant bezogen auf die Längsrichtung (128) angeordnet oder ausgebildet sind.

12. Implantationsset (204) umfassend ein modulares Implantatset (206) mit mindestens zwei unterschiedlichen Implantatteilen (20a, 20b, 20c; 120a, 120b, 120c; 120a', 120b', 120c') und ein medizinisches Instrumentarium (42; 142), insbesondere zum Implantieren eines Hüftgelenkschafts (20a, 20b, 20c; 120a, 120b, 120c; 120a', 120b', 120c'), umfassend ein Raspelinstrument (144) mit einem eine Längsrichtung (128) definierenden Raspelschaft (146), wobei am Raspelschaft (146) mindestens zwei Tiefenanschläge (152a, 152b, 152c) angeordnet oder ausgebildet sind, wobei jeder der mindestens zwei Tiefenanschläge (152a, 152b, 152c) mindestens eine Tiefenanschlagfläche (154a, 154b, 154c) umfasst, **dadurch gekennzeichnet, dass** die durch die mindestens zwei Tiefenanschläge (152a, 152b, 152c) definierten Tiefenanschlagflächen (154a, 154b, 154c) unterschiedliche Oberflächenstrukturen aufweisen oder unterschiedlich eingefärbt sind, dass die mindestens zwei unterschiedlichen Implantatteile (120a, 120b, 120c; 120a', 120b', 120c') jeweils ein Kennzeichnungselement (200a, 200b, 200c; 200a', 200b', 200c') umfassen, dass sich die Kennzeichnungselemente (200a, 200b, 200c; 200a', 200b', 200c') der mindestens zwei unterschiedlichen Implantatteile (120a, 120b, 120c; 120a', 120b', 120c') voneinander unterscheiden und dass jedem Kennzeichnungselement (200a, 200b, 200c; 200a', 200b', 200c') der mindestens zwei unterschiedlichen Implantatteile (120a, 120b, 120c; 120a', 120b', 120c') einer der mindestens zwei Tiefenanschläge (154a, 154b, 154c) zugeordnet ist.

13. Implantationsset nach Anspruch 13, **dadurch gekennzeichnet, dass** sich die Kennzeichnungselemente (200a, 200b, 200c; 200a', 200b', 200c') der mindestens zwei unterschiedlichen Implantatteile (120a, 120b, 120c; 120a', 120b', 120c') farblich, aufgrund ihrer Oberflächenstruktur und/ oder durch ihre Position am mindestens einen Implantatteil (120a, 120b, 120c; 120a', 120b', 120c') voneinander unterscheiden.

14. Implantationsset nach Anspruch 13, **dadurch gekennzeichnet, dass** die Kennzeichnungselemente (200a', 200b', 200c') der mindestens zwei unterschiedlichen Implantatteile (120a', 120b', 120c') in Form geometrischer Flächenbereiche (206a', 206b', 206c') ausgebildet sind.

15. Implantationsset nach Anspruch 13 oder 14, **dadurch gekennzeichnet, dass** die Kennzeichnungselemente (200a, 200b, 200c) mit den mindestens zwei unterschiedlichen Implantatteilen (120a, 120b, 120c) lösbar verbindbar ausgebildet oder unlösbar angeordnet oder ausgebildet sind.

## Claims

1. Medical instrumentation (42; 142), in particular for implanting a hip joint stem (20a, 20b, 20c; 120a, 120b, 120c; 120a', 120b', 120c'), comprising a rasping instrument (44; 144) having a rasp stem (46; 146) defining a longitudinal direction (28; 128), wherein the rasp stem (46; 146) has at least two depth stops (152a, 152b, 152c) arranged or formed thereon, wherein at least two of the at least two depth stops (152a, 152b) comprise at least one groove (192a, 192b), **characterized in that** the grooves (192a, 192b) on the rasp stem (146) extend parallel or essentially parallel to an end face (196) of the rasp stem (146) pointing in a proximal direction, **in that** the instrumentation comprises a coupling element (166) arranged or formed on the rasp stem (146) for coupling the rasp stem (146) with a handle element, **in that** the coupling element (166) is arranged or formed extending from the end face (196) pointing in a proximal or in an essentially proximal direction, and **in that** the depth stop faces (154a, 154b, 154c) defined by the at least two depth stops (152a, 152b, 152c) have different surface textures or are differently coloured.

2. Medical instrumentation in accordance with claim 1, **characterized in that** each of the at least two depth stops (152a, 152b, 152c) comprises at least one depth stop face (154a, 154b, 154c).

3. Medical instrumentation in accordance with claim 2, **characterized in that** the end face (196) of the rasp stem (146) pointing in a proximal direction comprises the at least one depth stop face (154c).

4. Medical instrumentation in accordance with any one of the preceding claims, **characterized in that** the at least two depth stops (152a, 152b, 152c) are arranged or formed in spaced-apart relationship to each other.

5. Medical instrumentation in accordance with any one of the preceding claims, **characterized in that** at least two of the at least two depth stops (152a, 152b) comprise at least one groove (192a, 192b).

6. Medical instrumentation in accordance with any one of the preceding claims, **characterized in that**
a) the at least one groove (192a, 192b) defines a groove longitudinal direction and/or extends in a straight line or in an essentially straight line,
and/or
b) the at least one groove (192a, 192b) is formed on a side face (180) of the rasp stem (146) that points in a direction transversely to the longitudinal direction.

7. Medical instrumentation in accordance with claim 2 or any one of claims 3 to 6, **characterized in that** the at least one groove (192a, 192b) comprises at least one groove side face (194a, 194b) and **in that** the at least one groove side face (194a, 194b) defines the at least one depth stop face (154a, 154b).

8. Medical instrumentation in accordance with claim 2 or any one of claims 3 to 7, insofar as they refer directly or indirectly back to claim 2, **characterized in that** the at least one depth stop face (154a, 154b, 154c) is planar.

9. Medical instrumentation in accordance with claim 2 or any one of claims 3 to 8, insofar as they refer directly or indirectly back to claim 2, **characterized in that** the at least one depth stop face (154a, 154b, 154c) of one of the at least two depth stops (152a, 152b, 152c) extends parallel or essentially parallel to the at least one depth stop face (154a, 154b, 154c) of another one of the at least two depth stops (152a, 152b, 152c).

10. Medical instrumentation in accordance with claim 2 or any one of claims 3 to 9, insofar as they refer directly or indirectly back to claim 2, **characterized in that** at least two depth stop faces (154a, 154b, 154c) define depth stop face planes (158a, 158b, 158c) which extend transversely, in particular perpendicularly, to the longitudinal direction (128).

11. Medical instrumentation in accordance with any one of claims 2 to 10, **characterized in that**
a) the rasp stem (146) is of symmetrical or essentially symmetrical configuration with respect to a plane of symmetry (56) which runs transversely, in particular perpendicularly, to at least one of the depth stop faces (154a, 154b, 154c),
and/or
b) there are provided three depth stop faces (154a, 154b, 154c), these being arranged or formed in equidistant or essentially equidistant relation with respect to the longitudinal direction (128).

12. Implantation set (204), comprising a modular implant set (206), including at least two different implant parts (20a, 20b, 20c; 120a, 120b, 120c; 120a', 120b', 120c'), and medical instrumentation (42; 142), in particular for implanting a hip joint stem (20a, 20b, 20c; 120a, 120b, 120c; 120a', 120b', 120c'), comprising a rasping instrument (144) having a rasp stem (146) defining a longitudinal direction (128), wherein the rasp stem (146) has at least two depth stops (152a, 152b, 152c) arranged or formed thereon, wherein each of the at least two depth stops (152a, 152b, 152c) comprises at least one depth stop face (154a, 154b, 154c), **characterized in that** the depth stop faces (154a, 154b, 154c) defined by the at least two depth stops (152a, 152b, 152c) have different surface textures or are differently coloured, **in that** the at least two different implant parts (120a, 120b, 120c; 120a', 120b', 120c') each comprise an identification element (200a, 200b, 200c; 200a', 200b', 200c'), **in that** the identification elements (200a, 200b, 200c; 200a', 200b', 200c') of the at least two different implant parts (120a, 120b, 120c; 120a', 120b', 120c') are different from each other, and **in that** each identification element (200a, 200b, 200c; 200a', 200b', 200c') of the at least two different implant parts (120a, 120b, 120c; 120a', 120b', 120c') is associated with one of the at least two depth stops (154a, 154b, 154c),

13. Implantation set in accordance with claim 13, **characterized in that** the identification elements (200a, 200b, 200c; 200a', 200b', 200c') of the at least two different implant parts (120a, 120b, 120c; 120a', 120b', 120c') are different from each other in respect of their colour schemes, on the basis of their surface textures and/or by their positions on the at least one implant part (120a, 120b, 120c; 120a', 120b', 120c').

14. Implantation set in accordance with claim 13, **characterized in that** the identification elements (200a', 200b', 200c') of the at least two different implant parts (120a', 120b', 120c') are configured in the form of geometrical surface portions (206a', 206b', 206c').

15. Implantation set in accordance with claim 13 or 14, **characterized in that** the identification elements (200a, 200b, 200c) are configured such that they are releasably connectable to the at least two different implant parts (120a, 120b, 120c) or such that they are arranged or formed in non-releasable relationship therewith.

## Revendications

1. Instrumentation médical (42 ; 142), en particulier pour l'implantation d'une tige fémorale (20a, 20b, 20c ; 120a, 120b, 120c ; 120a', 120b', 120c'), comprenant un instrument formant rugine (44 ; 144) pourvu d'une tige de rugine (46 ; 146) définissant une direction longitudinale (28 ; 128), dans lequel au moins deux butées de profondeur (152a, 152b, 152c) sont agencées ou formées sur la tige de rugine (46 ; 146), dans lequel au moins deux desdites au moins deux butées de profondeur (152a, 152b) comportent au moins une rainure (192a, 192b), **caractérisé en ce que** les rainures (192a, 192b) s'étendent sur la tige de rugine (146) parallèlement ou sensiblement parallèlement à une surface d'extrémité (196) de la tige de rugine (146), laquelle surface pointe dans la direction proximale, **en ce que** l'instrumentation comporte un élément d'accouplement (166) agencé ou formé sur la tige de rugine (146) et destiné à accoupler la tige de rugine (146) à un élément de préhension, **en ce que** l'élément d'accouplement (166) est agencé ou formé de manière à faire saillie en pointant à partir de la surface d'extrémité (196) dans la direction proximale ou sensiblement dans la direction proximale et **en ce que** les surfaces de butée de profondeur (154a, 154b, 154c) définies par lesdites au moins deux butées de profondeur (152a, 152b, 152c) présentent différentes structures superficielles ou sont colorées différemment.

2. Instrumentation médical selon la revendication 1, **caractérisé en ce que** chacune desdites au moins deux butées de profondeur (152a, 152b, 152c) comporte au moins une surface de butée de profondeur (154a, 154b, 154c).

3. Instrumentation médical selon la revendication 2, **caractérisé en ce que** la surface d'extrémité (196) de la tige de rugine (146), laquelle surface pointe dans la direction proximale, comporte ladite au moins une surface de butée de profondeur (154c).

4. Instrumentation médical selon l'une quelconque des revendications précédentes, **caractérisé en ce que** lesdites au moins deux butées de profondeur (152a, 152b, 152c) sont agencées ou formées à distance les unes des autres.

5. Instrumentation médical selon l'une quelconque des revendications précédentes, **caractérisé en ce qu'**au moins deux desdites au moins deux butées de profondeur (152a, 152b) comportent au moins une rainure (192a, 192b).

6. Instrumentation médical selon l'une quelconque des revendications précédentes, **caractérisé en ce que**
a) ladite au moins une rainure (192a, 192b) définit une direction longitudinale de rainure et/ou s'étend en ligne droite ou sensiblement en ligne droite
et/ou
b) ladite au moins une rainure (192a, 192b) est formée sur une surface latérale (180) de la tige de rugine (146), laquelle surface pointe dans une direction transversale à la direction longitudinale.

7. Instrumentation médical selon la revendication 2 ou selon l'une quelconque des revendications 3 à 6, **caractérisé en ce que** ladite au moins une rainure (192a, 192b) comporte au moins une surface latérale de rainure (194a, 194b) et **en ce que** ladite au moins une surface latérale de rainure (194a, 194b) définit ladite au moins une surface de butée de profondeur (154a, 154b).

8. Instrumentation médical selon la revendication 2 ou selon l'une quelconque des revendications 3 à 7, dans la mesure où celles-ci sont directement ou indirectement rattachées à la revendication 2, **caractérisé en ce que** ladite au moins une surface de butée (154a, 154b, 154c) est plate.

9. Instrumentation médical selon la revendication 2 ou selon l'une des revendications 3 à 8, dans la mesure où celles-ci sont directement ou indirectement rattachées à la revendication 2, **caractérisé en ce que** ladite au moins une surface de butée de profondeur (154a, 154b, 154c) d'une desdites au moins deux butées de profondeur (152a, 152b, 152c) s'étend parallèlement ou sensiblement parallèlement à ladite au moins une surface de butée de profondeur (154a, 154b, 154c) d'une autre desdites au moins deux butées de profondeur (152a, 152b, 152c).

10. Instrumentation médical selon la revendication 2 ou selon l'une des revendications 3 à 9, dans la mesure où celles-ci sont directement ou indirectement rattachées à la revendication 2, **caractérisé en ce que** lesdites au moins deux surfaces de butée de profondeur (154a, 154b, 154c) définissent des plans de surfaces de butée de profondeur (158a, 158b, 158c), lesquels s'étendent transversalement, en particulier perpendiculairement, à la direction longitudinale (128).

11. Instrumentation médical selon l'une quelconque des revendications 2 à 10, **caractérisé en ce que**
a) la tige de rugine (146) est formée de manière symétrique ou sensiblement symétrique à un plan de symétrie (56), lequel s'étend transversalement, en particulier perpendiculairement, à au moins une des surfaces de butée de profondeur (154a, 154b, 154c),
et/ou
b) **en ce que** trois surfaces de butée de profondeur (154a, 154b, 154c) sont prévues, qui sont agencées ou sont formées de manière équidistante ou sensiblement équidistante par rapport à la direction longitudinale (128).

12. Kit d'implantation (204) comportant un kit d'implant modulaire (206) pourvu d'au moins deux parties d'implant (20a, 20b, 20c ; 120a, 120b, 120c ; 120a', 120b', 120c') différentes et un instrumentation médical (42 ; 142), en particulier pour l'implantation d'une tige fémorale (20a, 20b, 20c ; 120a, 120b, 120c ; 120a', 120b', 120c'), comportant un instrument formant rugine (144) pourvu d'une tige de rugine (146) définissant une direction longitudinale (128), dans lequel au moins deux butées de profondeur (152a, 152b, 152c) sont agencées ou formées sur la tige de rugine (146), dans lequel chacune desdites au moins deux butées de profondeur (152a, 152b, 152c) comporte au moins une surface de butée de profondeur (154a, 154b, 154c), **caractérisé en ce que** les surfaces de butée de profondeur (154a, 154b, 154c) définies par lesdites au moins deux butées de profondeur (152a, 152b, 152c) présentent des structures superficielles différentes ou sont colorées différemment, **en ce que** lesdites au moins deux parties d'implant (120a, 120b, 120c ; 120a', 120b', 120c') différentes comportent chacune un élément d'identification (200a, 200b, 200c ; 200a', 200b', 200c'), **en ce que** les éléments d'identification (200a, 200b, 200c ; 200a', 200b', 200c') desdites au moins deux parties d'implant (120a, 120b, 120c ; 120a', 120b', 120c') différentes sont différents les uns des autres et **en ce qu'**une desdites au moins deux butées de profondeur (154a, 154b, 154c) est associée à chaque élément d'identification (200a, 200b, 200c ; 200a', 200b', 200c') desdites au moins deux parties d'implant (120a, 120b, 120c ; 120a', 120b', 120c') différentes.

13. Kit d'implantation selon la revendication 13, **caractérisé en ce que** les éléments d'identification (200a, 200b, 200c ; 200a', 200b', 200c') desdites au moins deux parties d'implant (120a, 120b, 120c ; 120a', 120b', 120c') différentes se distinguent les uns des autres par leur couleur, sur la base de leur structure superficielle et/ou par leur position sur au moins une partie d'implant (120a, 120b, 120c ; 120a', 120b', 120c').

14. Kit d'implantation selon la revendication 13, **caractérisé en ce que** les éléments d'identification (200a', 200b', 200c') desdites au moins deux parties d'implant (120a', 120b', 120c') différentes sont réalisés sous la forme de zones superficielles géométriques (206a', 206b', 206c').

15. Kit d'implantation selon la revendication 13 ou 14, **caractérisé en ce que** les éléments d'identification (200a, 200b, 200c) sont agencés de manière à pouvoir être reliés de manière libérable auxdites au moins deux parties d'implant (120a, 120b, 120c) différentes ou sont agencés ou formés de manière non libérable.
